# EUROPÄISCHE PATENTSCHRIFT

(11) **EP 2 689 835 B1**
(45) Veröffentlichungstag und Bekanntmachung des Hinweises auf die Patenterteilung: **08.05.2019**
(21) Anmeldenummer: 12178132.2
(22) Anmeldetag: 26.07.2012
(51) Int. Cl.: B01J 13/08, A01N 25/28, A61K 8/11, C09B 67/00, C11D 3/50

(54) **Duftölverkapselung**
Aromatic oil encapsulation
Encapsulage d'huile parfumée

(43) Veröffentlichungstag der Anmeldung: 29.01.2014
(73) Patentinhaber: Papierfabrik August Koehler SE, 77704 Oberkirch (DE); Symrise AG, 37603 Holzminden (DE)
(72) Erfinder: Bertram, Ralf, 37602 Holzminden (DE); Ott, Patrick, 37603 Holzminden-Silberborn (DE); Kühne, Lutz, 77797 Ohlsbach (DE); Meier, Claudia, 77839 Lichtenau (DE); Schroeder, Julien, 67000 Strasbourg (FR); Mahler, Stephan, 77694 Kehl (DE); Jurisch, Claus, 77770 Durbach (DE)
(74) Vertreter: Letzelter, Felix Phillip

(56) Entgegenhaltungen:
- EP-A1- 0 321 750
- DE-A1- 3 818 712

## Beschreibung

Die vorliegende Erfindung betrifft Mikrokapseln, die sich durch ein besonders günstiges Freisetzungsverhalten des in den Mikrokapseln eingeschlossenen Kernmaterials auszeichnen. Die vorliegende Erfindung betrifft darüber hinaus eine Suspension solcher Mikrokapseln in einer Flüssigkeit sowie deren Verwendung als Bestandteil von Flüssigwaschmitteln, Weichspülern, Reinigungsmitteln, Waschpulvern, Duschgelen, Shampoos, Deodorants und/oder Body-Lotions.

In vielen Fällen ist es wünschenswert, dass Mikrokapseln den in Form eines hydrophoben Kernmaterials eingeschlossenen Bestandteil unter genau definierten Bedingungen freisetzen. So ist es beispielsweise bei Mikrokapseln, die Duftsubstanzen enthalten, notwendig, dass die Mikrokapseln die meist sehr oxidationsempfindlichen Duftstoffe während der Lagerung stabil einschließen und dass erst im Augenblick der gewünschten Duftentwicklung ein durch mechanische Einwirkung verursachtes Aufbrechen der Mikrokapseln erfolgt.

Viele Artikel des täglichen Lebens wie beispielsweise Reinigungsmittel, Weichspüler, Waschpulver, Flüssigwaschmittel, Duschgele, Shampoos, Deodorants, Bodylotion etc. sind heute mit Riechstoffen bzw. Riechstoffmischungen parfümiert. Sehr häufig kommt es zu Wechselwirkungen der Riechstoffe mit anderen Bestandteilen der Formulierung oder zu einem vorzeitigen Abdampfen der leichter flüchtigen Komponenten einer Parfümierung. Dies führt in der Regel dazu, dass sich der Dufteindruck der Parfümierung verändert oder vollständig verschwindet.
Die Mikroverkapselung solcher Riechstoffmischungen bietet die Möglichkeit, Wechselwirkungen im parfümierten Produkt oder das Abdampfen der leicht flüchtigen Inhaltsstoffe zu verringern bzw. vollständig zu verhindern.

Üblicherweise werden Kern/Schale-Mikrokapseln durch das feine Dispergieren des Kernmaterials in einer Wasserphase hergestellt. Anschließend wird bei den Koazervationsverfahren das Wandmaterial aus der die Öltropfen umgebenden Phase (Wasserphase) auf den Öltropfen abgeschieden. Die Größe der Öltropfen bestimmt daher direkt die Größe der späteren Kapselkerne. Sehr häufig werden bei solchen Koazervationen Aminoplaste als Wandmaterial eingesetzt. Diese Aminoplaste basieren sehr häufig auf der Kondensation von Melamin und Formaldehyd bzw. anderer Aminkomponenten oder Aldehyden. Verfahren zur Herstellung solcher Mikrokapseln sind hinreichend bekannt.

Solche Mikrokapseln werden bereits in Flüssigwaschmitteln und Weichspülern eingesetzt. In diesem Zusammenhang seien hier die Publikationen US 2010/40884 (Appleton); WO 2003/2699, WO 2006/121639, WO 2008/5693, US 2008/295256, US 2010/0279916 (Colgate Palmolive); WO 2006/131846 (Firmenich); Chimia 2011, 65, No.3 177-181 (Bône et al.), WO 2008/98387, WO 2009/100553 (Givaudan); WO 2010/060677, WO 2010/28907, WO 2010/43452 (Henkel); EP 1797946, EP 1797947, US 2009/258042 (International Flavours and Fragrances); WO 2009/047745, WO 2010/114753 (Procter & Gamble); EP 2204155 (Takasago); WO 2011/120772 (Unilever) genannt.

Das bedeutet, dass ein die Kapsel enthaltender Weichspüler bzw. ein die Kapsel enthaltendes Flüssigwaschmittel der damit behandelten Wäsche einen länger anhaltenden Duft verleihen soll, als wenn das Duftöl frei vorläge, bzw. beim Reiben oder Schütteln der Wäsche einen Duftschwall auslösen soil. Die Kapseln müssen möglichst gut im Textilgewebe hängen bleiben und bei mechanischer Beanspruchung brechen und das enthaltene Duftöl freisetzen. Die optimalen Verhältnisse zwischen Kapselgröße und Kapselstabilität werden durch praktische Anwendungstests ermittelt. Die enge Vernetzung, wie sie beispielsweise in Aminoplasten auf Basis Melamin und Formaldehyd vorliegt, ist die Ursache für die hohe Dichtigkeit dieser Kapselwand in tensidhaltigem wässrigem Medium (Weichspüler, Flüssigwaschmittel).

EP 0 321 750 A1 offenbart Mikrokapseln mit einer Teilchengrößenverteilung, die zwei Maxima aufweist, wobei das Hauptmaximum der Teilchengröße bei etwa 5 µm liegt und ein Nebenmaximum der Teilchengrößenverteilung bei etwa 0.7 µm liegt und wobei das von den Mikrokapseln, deren Teilchengröße ≤ 1/4 der Teilchengröße des Hauptmaximums, d.h. etwa 1/4^{∗}5=1,25 µm beträgt, eingenommene Volumen etwa 25% des Gesamtvolumens der Mikrokapseln beträgt.

Es hat sich gezeigt, dass die bislang im Stand der Technik vorgeschlagenen Mikrokapseln eine für viele Anwendungen noch nicht hinreichende Lagerstabilität aufweisen. In diesem Zusammenhang sei auf die WO 01/49817 verwiesen, die Mikrokapsel-Zubereitungen beschreibt, die Mikrokapseln mit einem Kern aus einem hydrophoben Material, das wenigstens einen Duft- oder Riechstoff umfasst, beschreibt.

Insbesondere wird bei der Verwendung von solchen Mikrokapseln mit eingeschlossenen Duftölen in Flüssigwaschmitteln oder Weichspülern beobachtet, dass bei der Lagerung dieser Zusammensetzung über einen längeren Zeitraum ein erhebliches Freisetzen des Duftstoffes auftritt, was natürlich zu einer geringeren Duftfreisetzung am gewünschten Wirkungsort, nämlich der gewaschenen Wäsche, führt. Darüber hinaus hat sich bei den bislang im Stand der Technik beschriebenen Mikrokapseln bei Verkapselung von Duftölen gezeigt, dass Duftsubstanzen nicht im gewünschten Maße durch Reiben der behandelten Wäsche freigesetzt werden können.

Vor diesem Hintergrund lag der vorliegenden Erfindung die Aufgabe zugrunde, Mikrokapseln bereitzustellen, die eine höhere Lagerstabilität aufweisen. Insbesondere lag der vorliegenden Erfindung die Aufgabe zugrunde, Mikrokapseln bereitzustellen, die bei Lagerung in Form flüssiger Suspensionen der Mikrokapseln, wie z.B. in Waschmitteln oder Weichspülern, einer geringeren Freisetzung der Inhaltsstoffe, insbesondere in Form von Duftstoffen, unterliegen. Eine weitere Aufgabe der vorliegenden Erfindung bestand darin, Mikrokapseln mit einem hydrophoben Duftöl als Kernmaterial bereitzustellen, die nach Aufbringen auf Wäsche ein günstiges Freisetzungsverhalten der Duftöle bei Reiben der behandelten Wäsche zeigen.

Die erwähnte Aufgabe wird erfindungsgemäß gelöst durch Mikrokapseln mit einer volumenbezogenen Teilchengrößenverteilung, die mindestens zwei Maxima aufweist, wobei das Hauptmaximum der Teilchengröße im Bereich von 10 bis 50 µm liegt und ein Nebenmaximum der Teilchengrößenverteilung bei 1 bis 6 µm liegt und wobei das von den Mikrokapseln, deren Teilchengröße ≤ ein Viertel der Teilchengröße des Hauptmaximums beträgt, eingenommene Volumen ≥ 20% des Gesamtvolumens der Mikrokapseln beträgt.

Ein wesentliches Merkmal der erfindungsgemäßen Mikrokapseln ist also das volumenprozentuale Verhältnis der Teilchengrößenbereiche zueinander.

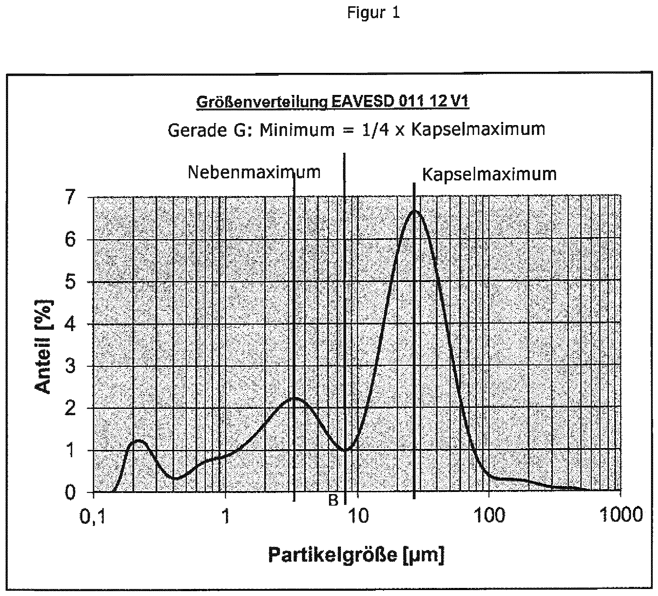

Dies soll im Folgenden anhand der in Figur 1 gezeigten Abbildung veranschaulicht werden:
Figur 1 zeigt eine graphische Auftragung der Teilchengröße von Mikrokapseln gegen den prozentualen Anteil der jeweiligen Teilchengröße. In Figur 1 schneidet die Gerade G den Graph an Punkt B bei einem Viertel der Teilchengröße des Hauptmaximums. Das links von diesem Punkt B liegende Kapselvolumen beträgt ≥ etwa 20% des Gesamtvolumens aller Kapseln. Diese Volumenverhältnisse kann man aus den Rohdaten der Volumenprozente entnehmen. Gebräuchlicher ist die umgekehrte Darstellung, indem man z. B. den mittleren Partikeldurchmesser angibt, unterhalb dem 90% des Teilchenvolumens liegt, der sogenannte d₉₀-Wert.

Überraschenderweise hat sich gezeigt, dass mit Flüssigwaschmittel bzw. Weichspüler, die die erfindungsgemäßen Kapseln enthalten, gewaschene Wäsche eine stärkere Duftfreisetzung nach Reibung aufweist als Wäsche, die mit Flüssigwaschmittel bzw. Weichspüler gewaschen wurde, welches die Melaminformaldehydkapseln gemäß dem Stand der Technik enthält. Anhand der physikalischen Daten wie Kapselmaximum, Wandstärke und freiem Ölgehalt unterscheiden sich die erfindungsgemäßen Kapseln nicht signifikant von den Mikrokapseln aus dem Stand der Technik. Es ist daher davon auszugehen, dass die angesprochene Teilchengrößenverteilung ursächlich für die verbesserte Duftfreisetzung ist.

Aus den Partikelgrößenverteilungen der erfindungsgemäßen Kapseldispersionen wird jedoch ersichtlich, dass sie deutlich mehr Feinanteil (kleine Kapseln und Polymergrieß) enthalten als die Wettbewerbskapseln. Die meisten Partikelgrößenverteilungskurven sind bimodal. Als Grenze zwischen Nebenpeak (Feinanteil) und Hauptkapselpeak wird das Kurvenminimum zwischen dem Hauptmaximum und dem Nebenmaximum definiert. Dem bei einigen Kapselchargen auftretenden 3. Peak im äußersten Feinbereich wird keine Bedeutung beigemessen. Sofern ein Minimum zwischen Haupt- und Nebenpeak vorhanden ist, liegt es zwischen 25% und 28,5% des Durchmessers des Hauptmaximums. Um auch bei monomodalen Kapseln eine Aussage über das Verhältnis von Feinanteil (kleine Kapseln und Polymergrieß) zur Hauptkapsel vornehmen zu können, wird die Grenze bei einem Teilchendurchmesser zwischen 25% und 28,5% des Kurvenmaximums definiert.

Mithilfe eines Partikelgrößenmessgeräts, welches Einzelfotografien aller Teilchen einer Probe aufnimmt, deren Durchmesser bestimmt und die Daten statistisch auswertet, kann man feststellen, dass es sich bei den Partikeln im Grenzbereich zwischen Haupt- und Nebenmaximum vorwiegend um Mikrokapseln handelt. Auch das Nebenmaximum besteht überwiegend aus Mikrokapseln. Die Partikelgröße des sogenannten Polymergrießes, der als Nebenprodukt bei dieser Art der Mikroverkapselung entsteht, liegt überwiegend bei < 3 µm. Genauere Aussagen über die Mengenverhältnisse zwischen kleinen Kapseln und Polymergrieß können nicht gemacht werden.

Eine genau belegbare Erklärung, warum bei den meisten Partikelgrößenverteilungskurven eine Bimodalität eintritt, ist nicht bekannt. Eine häufige Erklärung ist, dass Agglomerationsvorgänge der kleinen Kapseln dazu führen. Eine Ölemulsion vor der Verkapselung zeigt eine wesentlich schwächer ausgeprägte Bimodalität in ihrer Partikelgrößenverteilungskurve.

Insgesamt führt das bei den erfindungsgemäßen Kapseln typischerweise vorliegende Gemisch aus großen und kleinen Kapseln, Agglomeraten und Polymergrieß zu einer besseren Wirkung der Kapsel in den Endanwendungen. Dies ist ein stärkerer Duftschwall nach Reibung eines mit Flüssigwaschmittel bzw.

Weichspüler, die die erfindungsgemäßen Kapseln enthalten, behandelten Wäschestücks. Ohne an eine Theorie gebunden sein zu wollen, vermuten die Erfinder, dass das Vorliegen kleinerer Teilchen ein Aufbrechen von Kapseln, die ein hydrophobes Duftöl enthalten durch Reiben der Wäsche vereinfachen.

Es ist nicht möglich, die Menge an verkapseltem Duftöl in einem gewaschenen Wäschestück exakt quantitativ zu ermitteln, da man die darin hängenden Mikrokapseln nicht quantitativ abtrennen kann, bzw. die Mikrokapseln in der Wäsche nicht quantitativ zerstören kann.

In einer besonders bevorzugten Ausführungsform zeichnen sich die erfindungsgemäßen Mikrokapseln dadurch aus, dass das von den Mikrokapseln, deren Teilchengröße ≤ ein Viertel der Teilchengröße des Hauptmaximums beträgt, eingenommene Volumen ≥ etwa 22%, insbesondere ≥ etwa 25% des Gesamtvolumens der Mikrokapseln beträgt.

Es hat sich gezeigt, dass besonders gute Ergebnisse erzielt werden, wenn die erfindungsgemäßen Mikrokapseln so ausgestaltet sind, dass das Hauptmaximum der Teilchengrößenverteilung bei 15 bis 40 µm liegt und ein Nebenmaximum der Teilchengrößenverteilung bei 1 bis 6 µm, insbesondere bei 1,5 bis 4 µm, stärker bevorzugt bei 2 bis 3,5 µm liegt.

In einer besonders bevorzugten Ausführungsform sind die erfindungsgemäßen Mikrokapseln so ausgestaltet, dass das von den Mikrokapseln, deren Teilchengröße ≤ 6 Mikrometer beträgt, eingenommene Volumen ≥ etwa 20% des Gesamtvolumens der Mikrokapseln beträgt. In einer stärker bevorzugten Ausführungsform sind die erfindungsgemäßen Mikrokapseln so ausgestaltet, dass das von den Mikrokapseln, deren Teilchengröße ≤ 6 Mikrometer beträgt, eingenommene Volumen ≥ etwa 22%, insbesondere ≥ etwa 25% des Gesamtvolumens der Mikrokapseln beträgt.

Als Kapselwandmaterial kann in den erfindungsgemäßen Mikrokapseln jedes Material eingesetzt werden, das zur Bildung von Mikrokapseln geeignet ist. Bevorzugt handelt es sich bei dem Kapselwandmaterial der Mikrokapseln um ein Aminoplast. In einer besonders bevorzugten Ausführungsform sind die erfindungsgemäßen Mikrokapseln so ausgestaltet, dass die Mikrokapseln eine Kapselwand aus einem methylierten Melamin-Formaldehydharz und/oder Harnstoff-Formaldehydharz und/oder Reaktionsprodukten von Aldehyden mit Thioharnstoff, N-Alkylharnstoff, Guanidin, Acetoguanamin, Benzoguanamin, Caprionoguanamin, Cyanamin, Dicyandiamid und/oder Alkyl-/Arylsulfonamid aufweisen.

Als Kernmaterial der erfindungsgemäßen Mikrokapseln kommt jedes Material infrage, das für den Einschluss in Mikrokapseln geeignet ist. Bevorzugt weisen die erfindungsgemäßen Mikrokapseln ein Kernmaterial aus mindestens einem im Wesentlichen wasserunlöslichen Material auf. In einer besonders bevorzugten Ausführungsform sind die erfindungsgemäßen Mikrokapseln so ausgestaltet, dass die Mikrokapseln ein Kernmaterial aufweisen, das ein hydrophobes Duftöl, ein Pestizid, ein Biozid, ein Insektizid, eine Substanz aus der Gruppe der Repellentien, einen kosmetischen Wirkstoff, einen Farbstoff oder Farbstoffgemische, Leuchtfarben, optische Aufheller, Lösungsmittel, Wachse, Silikonöle, Schmierstoffe, Monomere für Polymerisationen oder Polykondensationen, reaktive Kunststoffe, z. B. Klebstoffe für Ein- oder Mehrfachkomponenten, Lackbestandteile, Flammschutzmittel, Pigmentdispersionen in organischen Lösungsmitteln, Aromastoffe und/oder Agrochemikalien umfasst.

In einer bevorzugten Ausführungsform weisen die erfindungsgemäßen Mikrokapseln ein Kernmaterial in Form eines hydrophoben Duftöls auf, wobei dieses Kernmaterial ein oder mehrere Riechstoffe enthält, ausgewählt aus der Gruppe bestehend aus:
- Extrakte aus natürlichen Rohstoffen wie Etherische Öle, Concretes, Absolues, Resine, Resinoide, Balsame, Tinkturen wie z.B. Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl; Fichtennadelöl; Galbanumöl; Galbanumresin; Geraniumöl; Grapefruitöl; Guajakholzöl; Gurjunbalsam; Gurjunalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepresst; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnussöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierte Inhaltsstoffe;
- Einzel-Riechstoffe aus der Gruppe der Kohlenwasserstoffe, wie z.B. 3-Caren; alpha-Pinen; beta-Pinen; alpha-Terpinen; gamma-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
- aliphatische Aldehyde und deren Acetale wie z.B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen;
- aliphatische Ketone und deren Oxime wie z.B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on;
- aliphatische schwefelhaltige Verbindungen wie z.B. 3-Methylthio-hexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
- aliphatische Nitrile wie z.B. 2-Nonensäurenitril; 2-Undecensäurenitril; 2-Tridecensäurenitril; 3,12-Tridecadiensäurenitril; 3,7-Dimethyl-2,6-octadiensäurenitril; 3,7-Dimethyl-6-octensäurenitril;
- Ester von aliphatischen Carbonsäuren wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat, insbesondere Ethyl-2-trans-4-cis-decadienoat; Methyl-2-octinat; Methyl-2-noninat; Allyl-2-isoamyloxyacetat; Methyl-3,7-dimethyl-2,6-octadienoat; 4-Methyl-2-pentyl-crotonat;
- Formiate, Acetate, Propionate, Isobutyrate, Butyrate, Isovalerianate, Pentanoate, Hexanoate, Crotonate, Tiglinate oder 3-Methyl-2-butenoate von acyclischen Terpenalkoholen wie z.B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol; 2,6-Dimethyl-2,5,7-octatrien-1-ol;
- acyclische Terpenaldehyde und -ketone wie z.B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton sowie deren Dimethyl- und Diethylacetale; insbesondere die Dimethyl- und Diethylacetale von Geranial, Neral, 7-Hydroxy-3,7-dimethyloctanal;
- Formiate, Acetate, Propionate, Isobutyrate, Butyrate, Isovalerianate, Pentanoate, Hexanoate, Crotonate, Tiglinate und 3-Methyl-2-butenoate von cyclischen Terpenalkoholen wie z.B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol;
- cyclische Terpenaldehyde und -ketone wie z.B. Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on;2-Methyl-4-(2,6,6-trimethyl-1-cyclohexen-1-yl)-2-butenal; Nootkaton; Dihydronootkaton; 4,6,8-Megastigmatrien-3-on; alpha-Sinensal; beta-Sinensal; acetyliertes Cedernholzöl (Methylcedrylketon);
- cyclische und cycloaliphatische Ether wie z.B. Cineol; Cedrylmethylether; Cyclododecylmethylether;1,1-Dimethoxycyclododecan; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
- cyclische und makrocyclische Ketone wie z.B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-l-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 7-Cyclohexadecen-1-on; (7/8)-Cyclohexadecen-1-on; 9-Cycloheptadecen-1-on; Cyclopentadecanon; Cyclohexadecanon;
- cycloaliphatische Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
- cycloaliphatische Ketone wie z.B. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
- Ester cyclischer Alkohole wie z.B. 2-tert-Butylcyclohexylacetat; 4-tert-Butylcyclohexylacetat; 2-tert-Pentylcyclohexylacetat; 4-tert-Pentylcyclohexylacetat; 3,3,5-Trimethylcyclohexylacetat; Decahydro-2-naphthylacetat;2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
- Ester cycloaliphatischer Alkohole wie z.B.1-Cyclohexylethylcrotonat;
- Ester cycloaliphatischer Carbonsäuren wie z.B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
- Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
- araliphatische Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
- aromatische und araliphatische Aldehyde wie z.B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 2-Methyl-3-(4-isobutylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl) propanal;
- aromatische und araliphatische Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon;2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl] ethanon; 5',6',7',8'-Tetrahydro-3',5',5',6',8',8'-hexamethyl-2-acetonaphthon;
- aromatische und araliphatische Carbonsäuren und deren Ester wie z.B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
- stickstoffhaltige aromatische Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin;2-(3-Phenylpropyl)pyridin; Indol; Skatol; 2-Methoxy-3-isopropylpyrazin; 2-Isobutyl-3-methoxypyrazin;
- Phenylether und Phenylester wie z.B. Estragol; Anethol; Eugenylmethylether; Isoeugenylmethylether; Diphenylether; beta-Naphthylmethylether; beta-Naphthylethylether; beta-Naphthylisobutylether; 1,4-Dimethoxybenzol; Eugenylacetat; p-Kresylphenylacetat;
- heterocyclische Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on; und
- Dihydrocumarin; Octahydrocumarin; Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 4-Methyl-1,4-decanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin;

Ein für die Einsetzbarkeit der Mikrokapseln wichtiges Kriterium ist das Gewichtsverhältnis von Kernmaterial zu Kapselwandmaterial. Während einerseits ein möglichst hoher Anteil an Kernmaterial angestrebt wird, um einen möglichst hohen Nutzwert der Kapseln zu ermöglichen, ist es auf der anderen Seite notwendig, dass die Kapsel einen noch hinreichenden Anteil an Kapselwandmaterial aufweisen, damit die Stabilität der Kapseln gewährleistet ist.

Erfindungsgemäß hat es ich als besonders vorteilhaft erwiesen, dass die Mikrokapseln so ausgestaltet sind, dass die Mikrokapseln ein Gewichtsverhältnis von Kernmaterial zu Kapselwandmaterial aufweisen, das bei etwa 50-90 zu 50-10, insbesondere bei etwa 70-80 zu 30-20 liegt.

Eine besonders geeignete Form des Einsatzes der erfindungsgemäßen Mikrokapseln besteht darin, sie in Form einer Suspension dem Endprodukt zuzumischen. Daher betrifft die vorliegende Erfindung auch eine Suspension der oben beschriebenen Mikrokapseln in einer Flüssigkeit, insbesondere in Wasser. Eine solche Suspension weist besonders vorteilhafte Eigenschaften auf, wenn sie so ausgestaltet ist, dass der Gewichtsanteil der Mikrokapseln bei etwa 20 bis 60 Gew.-%, insbesondere bei etwa 25 bis 50 Gew.-%, stärker bevorzugt bei etwa 30 bis 35 Gew.-% liegt.

Um ein Mischen einer solchen Suspension zu verhindern und somit eine hohe Lagerstabilität zu erzielen, hat es sich als vorteilhaft erwiesen, dass die Suspension eine Viskosität von 12 bis 1500 mPas aufweist. Um die gewünschte Viskosität der Suspension zu erhalten wird vorzugsweise ein Verdickungsmittel eingesetzt.

Besonders geeignet sind die erfindungsgemäßen Mikrokapseln zum Einschluss von hydrophoben Duftölen, die dann in verschiedenen Reinigungsprodukten eingesetzt werden können.

Die vorliegende Erfindung betrifft somit insbesondere auch die Verwendung der beschriebenen Mikrokapseln als Bestandteil von Flüssigwaschmitteln, Weichspülern, Reinigungsmitteln, Waschpulvern, Duschgelen, Shampoos, Deodorants und/oder Body-Lotions.

In einer bevorzugten Ausführungsform erfolgt die Verwendung so, dass bei der wie oben beschriebenen Suspension der erfindungsgemäßen Mikrokapseln in einer Flüssigkeit zunächst das Wasser insbesondere durch Sprühtrocknung entzogen wird und die so getrockneten Mikrokapseln dem Produkt zugesetzt werden.

Um die Teilchengrößenverteilung von Mikrokapseln bestimmen zu können, und daraus die Verhältnisse von Größenbereichen der Teilchen ermitteln zu können, wird im Sinne der vorliegenden Erfindung das folgende Verfahren eingesetzt: Die Messung der Partikelgrößenverteilung erfolgte mit Hilfe der Laserdiffraktometrie. Dazu wurde ein Mastersizer 2000 (Malvern Instruments, Worcestershire, UK) verwendet, der mit einer Flüssigdispergiereinheit Hydro 2000SM ausgestattet ist. Die Dispergiereinheit verfügt über einen mechanischen Rührer.
Zur Messung wurde eine ausreichende Menge der Probe mit Hilfe der Dispergiereinheit in Wasser dispergiert und vermessen.
Die Rohdaten wurden anschließend mit Hilfe der mitgelieferten Software Mastersizer 2000 Version 5.54 ausgewertet. Als optisches Modell zur Ermittlung der Partikelgröße wird von dieser Software die Mie-Theorie verwendet

Es hat sich gezeigt, dass der Einsatz von erfindungsgemäßen Mikrokapseln, in denen hydrophobe Duftöle eingekapselt sind, gegenüber den aus dem Stand der Technik bekannten Mikrokapseln zu deutlichen Vorteilen führt. Dies wurde insbesondere auch durch sensorische Tests gezeigt, die mit Wäsche durchgeführt wurden, die mit Duftsubstanzen enthaltenden Mikrokapseln gemäß der vorliegenden Erfindung bzw. mit Mikrokapseln aus dem Stand der Technik in Form eines die Mikrokapseln enthaltenden Weichspülers bzw. einer Flüssigwaschmittelformulierung behandelt wurde. Bei der sensorischen Bewertung wurde eine Geruchsbewertung des Wäschestücks ohne und mit Reibung vorgenommen und mit einer Notenskala bewertet.

Die oben beschriebene Erfindung wird im Folgenden anhand von Beispielen noch näher erläutert.

### Beispiel 1

### Herstellung einer erfindungsgemäßen Mikrokapsel

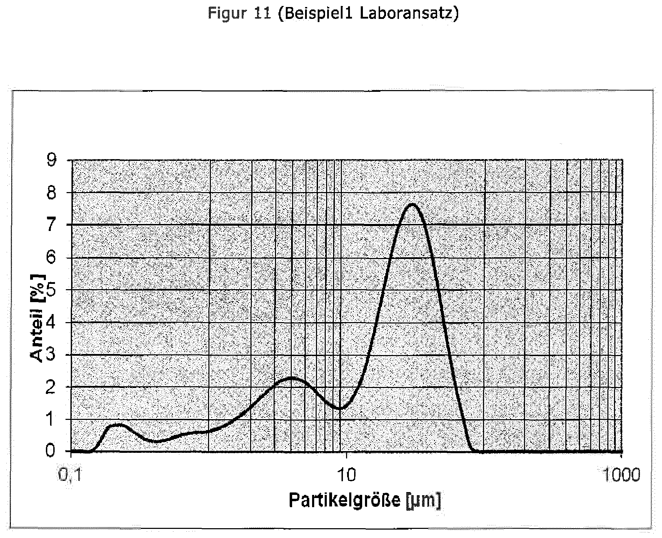

In einem zylindrischen 1I-Becherglas werden 28,95 g einer 40%-igen Lösung eines sulfonierten Melamin-Formaldehyd-Vorkondensats (z. B.: Melapret AAS 40 M, Fa. Melamin kemična tovarna d.d., Kočevje) bei ca. 22°C mit 146,50 g Wasser verdünnt. Unter Rühren mit einem Hochgeschwindigkeitsrührer (IKA Eurostar power control-visc 6000 mit Rührwerkzeug R1402 (Dissolverscheibe mit Präzisionswelle) bei 2400 U/min gibt man 32,47 g einer wässrigen Lösung eines kationisierten Melamin-Formaldehyd-Vorkondensats (z. B. Melapret KMS 30 N, Fa. Melamin kemična tovarna d.d., Kočevje), die durch Auflösen von 7,90 g einer 30%-igen Lösung desselben in 24,57 g Wasser erhalten wurde und rührt für 30 s. Zu dieser Lösung von ca. 22°C gibt man unmittelbar danach ohne Unterbrechung des Rührens bei weiterhin 2400 U/min 190,09 g eines nicht-wassermischbaren Duftöles (z. B. Tomcap, Fa. Symrise) von ebenfalls 22°C und emulgiert 30 min. Man erhält eine stabile Öl-in-Wasser-Emulsion, deren mittlere Teilchengröße von ca. 20 µm über Laserbeugung (Coulter LS 230, Optisches Modell nach Fraunhofer oder Malvern Mastersizer 2000m Optisches Modell nach Mie) bestimmt wird. Die Emulsion überführt man in einen geschlossenen 1 I-4-Halskolben mit Flügelrührer und Rückflusskühler, der in einem Wasserbad bei 30°C temperiert wird. Danach gibt man unter Rühren 7,44 g 20,5-ige Ameisensäure zu, wobei sich ein pH-Wert von ca. 3,8 einstellt. Zu dieser Emulsion gibt man 73 g einer 41,0%-igen Lösung eines methylierten Melamin-Formaldehyd-Vorkondensats (Wandbildnerharz, z. B.: Melapret NF 70 M, Fa. Melamin kemična tovarna d.d., Kočevje) und erhitzt innerhalb von 15 min auf 60°C. Die Rührgeschwindigkeit ist so anzupassen, dass eine gute Durchmischung gewährleistet ist. Bei Erreichen der 60°C verdünnt man mit 57,60 g Wasser. Nach weiteren 15 min gibt man 21,32 g Wandbildnerharzlösung zu. Im Anschluss wird durch Zugabe von 20,5%-iger Ameisensäure der pH-Wert wieder auf den vor der Zugabe gemessenen Wert eingestellt. Man lässt den Ansatz für 3,5 h bei 60°C weiterrühren. Nach Beendigung der 4-stündigen Reaktionszeit wird die Heizung des Wasserbads abgestellt und die Dispersion unter Rühren auf Raumtemperatur abgekühlt. Durch Zugabe von 4 g 12,5%-ige Ammoniaklösung stellt man den pH-Wert von 8,5 ein und senkt dadurch den Gehalt an freiem Formaldehyd auf unter 1500 ppm.
Man erhält eine Kapseldispersion mit einer bimodalen (strenggenommen trimodalen) Partikelgrößenverteilungskurve (Fig. 11) und einer mittleren Teilchengröße von 20,1 µm (D(4,3)-Wert, Malvern Mastersizer 2000). Der Feinananteil in dieser Kapsel beträgt entsprechend der in Fig. 1 dargestellten Vorgehensweise 30,1%.

### II Beschreibung der sensorischen Test zur Beurteilung der Kapseln

### Zusammensetzung Flüssigwaschmittel:

Zur sensorischen Bewertung der Kapseln wurden die Dispersionen mit einem Parfümölgehalt von ca. 25% in eine Flüssigwaschmittelformulierung (ca. 29% waschaktive Substanzen) in einer Konzentration von 0,2% eingearbeitet. Anschließend wurden in einer marktüblichen europäischen Waschmaschine 40 g dieser Formulierung auf 2 kg Wäsche verwaschen.

### Zusammensetzung Weichspüler:

Zur sensorischen Bewertung der Kapseln wurden die Dispersionen mit einem Parfümölgehalt von ca. 25% in eine Weichspülerformulierung (ca. 15% Esterquat)) in einer Konzentration von 0,2% eingearbeitet. Anschließend wurden in einer marktüblichen europäischen Waschmaschine 20 g dieser Formulierung auf 2 kg Wäsche verwaschen.

### Durchführung der sensorischen Bewertung:

Zur sensorischen Bewertung wurden die Proben anonymisiert und jedes Muster war zweimal vorhanden. Es wurden jeweils max. 4 Proben gleichzeitig bewertet. Das erste Muster war immer unparfümiert. Eine weitere unparfümierte Probe befand sich unter den anonymisierten Mustern. Das Expertenpanel bestand aus min. 12 Probanden.

Die Geruchsbewertung wird an dem Wäschestück ohne und mit Reibung vorgenommen und nach einer Notenskala bewertet (Je höher die Zahl, desto intensiver der wahrnehmbare Duft).

### III Vergleich von erfindungsgemäßen Mikrokapseln mit Mikrokapseln gemäß dem Stand der Technik

Es wurde eine Reihe von Versuchen durchgeführt, bei denen erfindungsgemäße Mikrokapseln in Bezug auf die relevanten Eigenschaften mit Mikrokapseln verglichen werden, wie sie im Stand der Technik beschrieben werden.

Dazu wurden in den Beispielen 2, 4, 6, 8 und 10 erfindungsgemäße Kapseln mit verschiedenen hydrophoben Duftölen hergestellt. Bei den Beispielen 1, 3, 5 und 7 handelt es sich um Vergleichsbeispiele, für die kommerziell erhältliche Mikrokapseln mit denselben verkapselten Duftölen herangezogen wurden. Dabei wurden folgende kommerziell erhältliche Kapseln eingesetzt:
Symcap®Tomcap FS ST (Mat.-Nr.: 338550)
Symcap® Fruit Rush FS ST (Mat.-Nr.: 359173), enthält Kapselöl W-Cap
Symcap® Extreme Power FS ST (Mat.-Nr.: 338698)
Symcap® Flower Burst FS ST (Mat.-Nr.: 609818) (Vertrieb der Kapseln durch Symrise AG, 37603 Holzminden, Deutschland)

Die Duftöle, die eingekapselt wurden, sind ebenfalls kommerziell erhältlich bei Symrise AG, 37603 Holzminden, Deutschland.
1) Kapselöl Tomcap (Mat.-Nr.: 266485), Vergleichsbeispiel
2) Kapselöl Tomcap (Mat.-Nr.: 266485), erfindungsgemäße Mikrokapsel
3) Kapselöl Flowercap (Mat.-Nr.: 369892), Vergleichsbeispiel
4) Kapselöl Flowercap (Mat.-Nr.: 369892), erfindungsgemäße Mikrokapsel
5) Kapselöl Extreme Power (Mat.-Nr.: 229831), Vergleichsbeispiel
6) Kapselöl Extreme Power (Mat.-Nr.: 229831), erfindungsgemäße Mikrokapsel
7) Kapselöl W-Cap (Mat.-Nr.: 358653), Vergleichsbeispiel
8) Kapselöl W-Cap (Mat.-Nr.: 358653), erfindungsgemäße Mikrokapsel

Um die Mikrokapseln aus dem Stand der Technik mit den erfindungsgemäßen Kapseln vergleichen zu können, wurden diese jeweils hinsichtlich Kapseldurchmesser, Kapselwandstärke und Teilchengrößenverteilung charakterisiert und sensorischen Tests unterworfen. Das Ergebnis wird in der folgenden Tabelle 1 wiedergegeben:

**Tabelle 1**

| Versuchsnummer | | 1 | 2-1 | 2-2 | 3 | 4 | 5 | 6 | 7 | 8 |
|---|---|---|---|---|---|---|---|---|---|---|
| Kapseldurchmesser* | µm | 26,0 | 23,3 | 22,9 | 17,0 | 20,9 | 19,8 | 18,6 | 16,8 | 21,5 |
| Kapselwandstärke** | nm | 260-350 | 130-180 | 170-200 | - | - | - | - | - | - |
| Kapselmaximum | µm | 28,5 | 26,5 | 26,5 | 20,1 | 20,1 | 21,6 | 21,6 | 18,9 | 21,6 |
| Nebenmaximum | µm | - | 3,1 | 3,6 | - | 2,9 | 2,0 | 2,9 | 1,7 | 2,9 |
| Feinanteil (Grenze 1/4 des Maximums) | % | 18,8 | 33,0 | 32,8 | 17,3 | 25,2 | 14,2 | 29,7 | 16,7 | 25,6 |
| Feinanteil (Grenze 1/3,5 des Maximums) | % | 20,7 | 33,0 | 32,8 | 17,3 | 25,2 | 14,7 | 31,0 | 16,9 | 26,8 |
| Freies Duftöl | % | 0,15 | 0,14 | 0,13 | 0,19 | 0,11 | 0,05 | 0,06 | 0,13 | 0,10 |
| Sensoriktest in Weichspüler ohne Reibung | Note | 0,08 | 0,28 | 0,17 | - | - | 0,12 | 0,09 | - | - |
| Sensoriktest in Weichspüler nach Reibung | Note | 3,54 | 3,88 | 3,85 | - | - | 2,43 | 2,81 | - | - |
| Sensoriktest in Flüssigwaschmittel ohne Reibung | Note | 0,03 | 0,29 | 0,12 | 0,86 | 0,25 | 0,06 | 0,11 | 0,25 | 0,10 |
| Sensoriktest in Flüssigwaschmittel nach Reibung | Note | 2,7 | 3,19 | 3,18 | 3,50 | 3,71 | 2,86 | 2,78 | 3,09 | 3,58 |
| Freies Duftöl nach 1 Woche 40°C in Weichspüler | % | 5,5 | 4 | 4 | 13,2 | 3,2 | 7 | 1 | 8,2 | 4,5 |
| Sensoriktest in Weichspüler ohne Reibung | Note | 0,15 | 0,63 | 0,32 | - | - | 0,40 | 0,14 | - | - |
| Sensoriktest in Weichspüler nach Reibung | Note | 2,17 | 3,80 | 3,59 | - | - | 1,69 | 2,76 | - | - |
| Freies Duftöl nach 1 Woche 40°C in Flüssigwaschmittel | % | 10 | 2 | 2 | - | 1,7 | - | 0,75 | - | 1,5 |
| Sensoriktest in Flüssigwaschmittel ohne Reibung | Note | 0,08 | 0,38 | 0,17 | 0,39 | 0,32 | 0,13 | 0,03 | 0,13 | 0,09 |
| Sensoriktest in Flüssigwaschmittel nach Reibung | Note | 2,29 | 3,64 | 3,07 | 2,71 | 3,39 | 2,13 | 2,91 | 2,91 | 3,56 |

| | | | | | | | | | | |
|---|---|---|---|---|---|---|---|---|---|---|
| * Mastersizer 2000, D(4,3)-Wert, volumenbezogener Mittelwert ** Bestimmung über Rasterelektronenmikroskopie von Kapseln, die bei tiefen Temperaturen zerstört wurden (Kryobruchverfahren), durch das Fraunhofer-Institut IAP, Potsdam-Golm. | | | | | | | | | | |

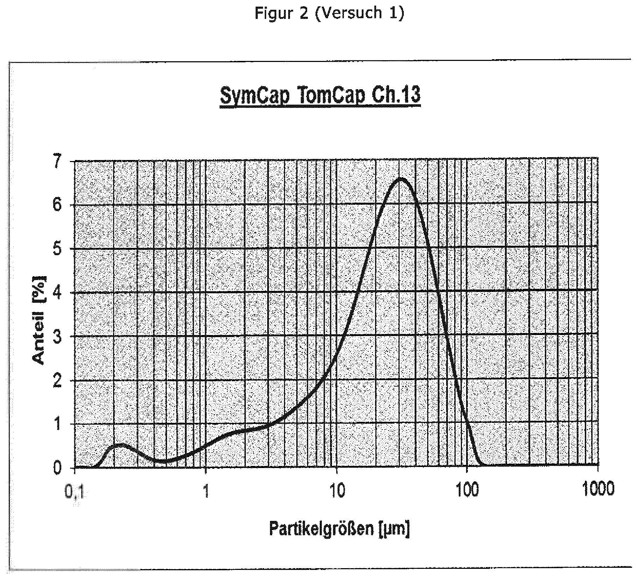

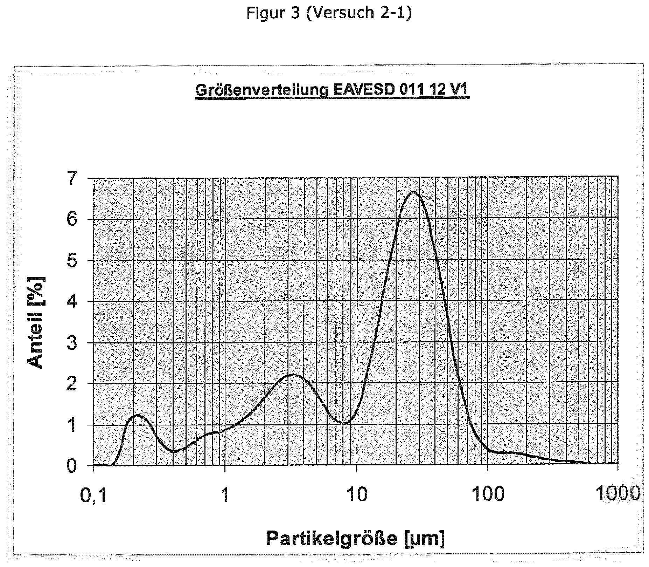

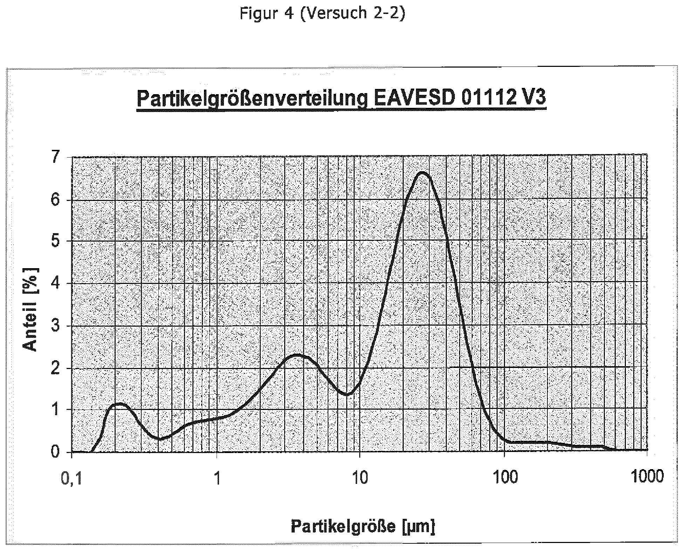

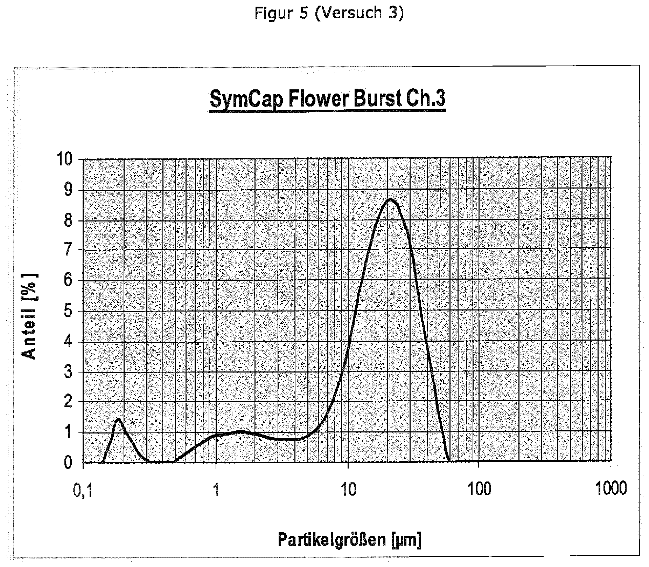

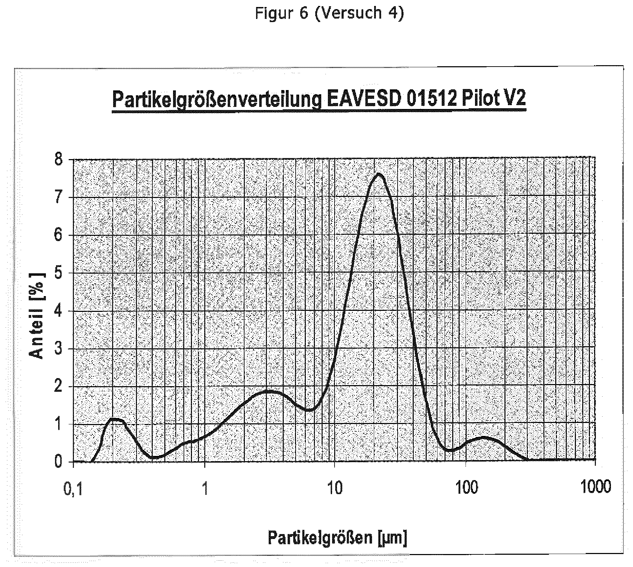

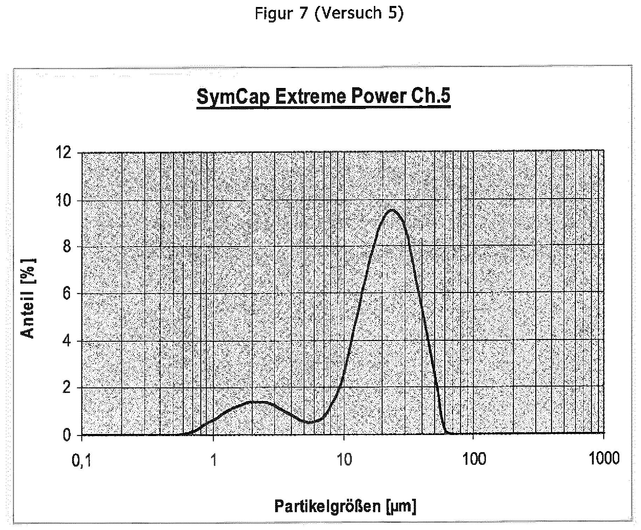

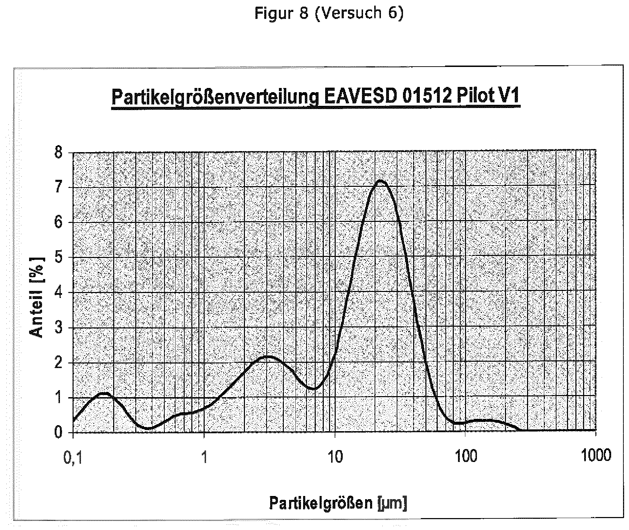

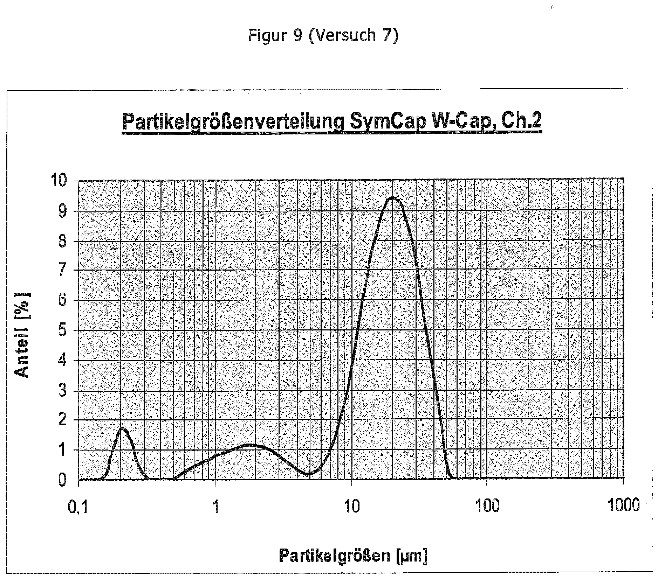

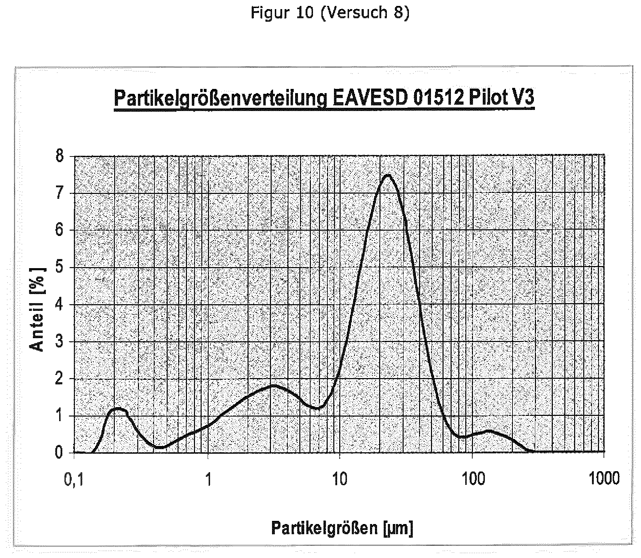

Die Teilchengrößenverteilung für die Beispiele 1 bis 8 wird in den Figuren 2 bis 10 wiedergegeben.

## Patentansprüche

1. Mikrokapseln mit einer Teilchengrößenverteilung, die mindestens zwei Maxima aufweist, wobei das Hauptmaximum der Teilchengröße im Bereich von 10-50 µm liegt und ein Nebenmaximum der Teilchengrößenverteilung bei 1 bis 6 µm liegt und wobei das von den Mikrokapseln, deren Teilchengröße ≤ ¼ der Teilchengröße des Hauptmaximums beträgt, eingenommene Volumen ≥ 20% des Gesamtvolumens der Mikrokapseln beträgt.

2. Mikrokapseln gemäß Anspruch 1,
**dadurch gekennzeichnet, dass**
das von den Mikrokapseln, deren Teilchengröße ≤ ¼ der Teilchengröße des Hauptmaximums beträgt, eingenommene Volumen ≥ 22%, insbesondere ≥ 25% des Gesamtvolumens der Mikrokapseln beträgt.

3. Mikrokapseln gemäß Anspruch 1 oder 2,
**dadurch gekennzeichnet, dass**
das Hauptmaximum der Teilchengrößenverteilung bei 15 bis 40 µm liegt und ein Nebenmaximum der Teilchengrößenverteilung bei 1 bis 6 µm, insbesondere bei 1,5 bis 4 µm, stärker bevorzugt bei 2 bis 3,5 µm liegt.

4. Mikrokapseln gemäß mindestens einem der Ansprüche 1 bis 3,
**dadurch gekennzeichnet, dass**
das von den Mikrokapseln, deren Teilchengröße ≤ 6 Mikrometer beträgt, eingenommene Volumen ≥ 20%, insbesondere ≥ 22% des Gesamtvolumens der Mikrokapseln beträgt.

5. Mikrokapseln gemäß mindestens einem der Ansprüche 1 bis 4,
**dadurch gekennzeichnet, dass**
die Mikrokapseln eine Kapselwand aus einem Aminoplast aufweisen.

6. Mikrokapseln gemäß mindestens einem der vorhergehenden Ansprüche,
**dadurch gekennzeichnet, dass**
die Mikrokapseln eine Kapselwand aus einem methylierten Melamin-Formaldehydharz und/oder Harnstoff-Formaldehydharz und/oder Reaktionsprodukten von Aldehyden mit Thioharnstoff, N-Alkylharnstoff, Guanidin, Acetoguanamin, Benzoguanamin, Capronoguanamin, Cyanamin, Dicyandiamid und/oder Alkyl-/Arylsulfonamid aufweisen.

7. Mikrokapseln gemäß mindestens einem der vorhergehenden Ansprüche,
**dadurch gekennzeichnet, dass**
die Mikrokapseln ein Kernmaterial aus mindestens einem wasserunlöslichen Material aufweisen.

8. Mikrokapseln gemäß mindestens einem der vorhergehenden Ansprüche,
**dadurch gekennzeichnet, dass**
die Mikrokapseln ein Kernmaterial aufweisen, das ein hydrophobes Duftöl, ein Pestizid, ein Biozid, ein Insektizid, eine Substanz aus der Gruppe der Repellentien, einen kosmetischen Wirkstoff, einen Farbstoff oder Farbstoffgemische, Leuchtfarben, optische Aufheller, Lösungsmittel, Wachse, Silikonöle, Schmierstoffe, Monomere für Polymerisationen oder Polykondensationen, reaktive Kunststoffe, z. B. Klebstoffe für Ein- oder Mehrfachkomponenten, Lackbestandteile, Flammschutzmittel, Pigmentdispersionen in organischen Lösungsmitteln, Aromastoffe und/oder Agrochemikalien umfasst.

9. Mikrokapseln gemäß mindestens einem der vorhergehenden Ansprüche,
**dadurch gekennzeichnet, dass**
die Mikrokapseln ein Gewichtsverhältnis von Kernmaterial zu Kapselwandmaterial aufweisen, das bei 50-90 zu 50-10, insbesondere bei 70-80 zu 30-20 liegt.

10. Suspension von Mikrokapseln gemäß mindestens einem der Ansprüche 1 bis 8 in einer Flüssigkeit.

11. Suspension gemäß Anspruch 10,
**dadurch gekennzeichnet, dass**
der Gewichtsanteil der Mikrokapseln bei 20 bis 60 Gew.-%, insbesondere bei 25 bis 50 Gew.-%, stärker bevorzugt bei 30 bis 35 Gew.-% liegt.

12. Suspension gemäß mindestens einem der Ansprüche 10 oder 11,
**dadurch gekennzeichne**t, dass
die Suspension eine Viskosität im Bereich von 12 bis 1500 mPas aufweist.

13. Verwendung der Mikrokapseln gemäß einem der Ansprüche 1 bis 9 oder der Suspension gemäß mindestens einem der Ansprüche 10 bis 12 als Bestandteil von Flüssigwaschmitteln, Weichspülern, Reinigungsmitteln, Waschpulvern, Duschgelen, Shampoos, Deodorants und/oder Body-Lotions.

14. Verwendung der Mikrokapseln gemäß Anspruch 13 unter vorherigem Entfernen des Wassers aus der Suspension insbesondere durch Sprühtrocknen.

## Claims

1. Microcapsules having a particle size distribution which has at least two maxima, wherein the main maximum of the particle size is in the range from 10 - 50 µm and a secondary maximum of the particle size distribution is 1 to 6 µm, and wherein the volume taken up by the microcapsules the particle size of which amounts to ≤ ¼ of the particle size of the main maximum amounts to ≥ 20% of the total volume of the microcapsules.

2. Microcapsules according to Claim 1,
**characterised in that**
the volume taken up by the microcapsules the particle size of which amounts to ≤ ¼ of the particle size of the main maximum amounts to ≥ 22%, especially ≥ 25%, of the total volume of the microcapsules.

3. Microcapsules according to Claim 1 or Claim 2,
**characterised in that**
the main maximum of the particle size distribution is 15 to 40 µm and a secondary maximum of the particle size distribution is 1 to 6 µm, especially 1.5 to 4 µm, more preferably 2 to 3.5 µm.

4. Microcapsules according to at least one of Claims 1 to 3,
**characterised in that**
the volume taken up by the microcapsules the particle size of which amounts to ≤ 6 micrometres amounts to ≥ 20%, especially ≥ 22%, of the total volume of the microcapsules.

5. Microcapsules according to at least one of Claims 1 to 4,
**characterised in that**
the microcapsules have a capsule wall made of an aminoplast.

6. Microcapsules according to at least one of the preceding claims,
**characterised in that**
the microcapsules have a capsule wall made of a methylated melamine formaldehyde resin and/or urea formaldehyde resin and/or reaction products of aldehydes with thiourea, N-alkyl urea, guanidine, acetoguanamine, benzoguanamine, capronoguanamine, cyanamine, dicyandiamide and/or alkyl/aryl sulfonamide.

7. Microcapsules according to at least one of the preceding claims,
**characterised in that**
the microcapsules have a core material made of at least one water-insoluble material.

8. Microcapsules according to at least one of the preceding claims,
**characterised in that**
the microcapsules have a core material which comprises a hydrophobic fragrant oil, a pesticide, a biocide, an insecticide, a substance from the group of repellents, a cosmetic active ingredient, a dye or dye mixtures, luminescent paints, optical brighteners, solvents, waxes, silicone oils, lubricants, monomers for polymerisations or polycondensations, reactive plastics materials, e.g. adhesives for single components or multiple components, paint constituents, flameproofing agents, pigment dispersions in organic solvents, aromatic substances and/or agrochemicals.

9. Microcapsules according to at least one of the preceding claims,
**characterised in that**
the microcapsules have a weight ratio of core material to capsule wall material which is from 50 - 90 to 50 - 10, especially 70 - 80 to 30 - 20.

10. A suspension of microcapsules according to at least one of Claims 1 to 8 in a liquid.

11. A suspension according to Claim 10,
**characterised in that**
the proportion by weight of the microcapsules is 20 to 60 wt.%, especially 25 to 50 wt.%, more preferably 30 to 35 wt.%.

12. A suspension according to at least one of Claims 10 or 11,
**characterised in that**
the suspension has a viscosity in the range from 12 to 1500 mPas.

13. Use of the microcapsules according to one of Claims 1 to 9 or of the suspension according to at least one of Claims 10 to 12 as a component of liquid detergents, fabric conditioners, cleaning agents, washing powders, shower gels, shampoos, deodorants and/or body lotions.

14. Use of the microcapsules according to Claim 13 with prior removal of the water from the suspension, especially by spray-drying.

## Revendications

1. Microcapsules ayant une distribution de tailles de particules qui présente au moins deux maxima, où le maximum principal de la taille de particules est compris dans la plage de 10-50 µm et un maximum secondaire de la distribution de tailles de particules est situé aux environs de 1 à 6 µm et où le volume occupé par les microcapsules, dont la taille de particules est ≤ 1/4 de la taille de particules du maximum principal, est ≥ 20 % du volume total des microcapsules.

2. Microcapsules selon la revendication 1, **caractérisées en ce que** le volume occupé par les microcapsules, dont la taille de particules est ≤ 1/4 de la taille de particules du maximum principal, est ≥ 22 %, en particulier ≥ 25 % du volume total des microcapsules.

3. Microcapsules selon la revendication 1 ou 2, **caractérisées en ce que** le maximum principal de la distribution de tailles de particules est aux environs de 15 à 40 µm, et un maximum secondaire de la distribution de tailles de particules est situé aux environs de 1 à 6 µm, en particulier aux environs de 1,5 à 4 µm, de manière plus préférée aux environs de 2 à 3,5 µm.

4. Microcapsules selon au moins l'une des revendications 1 à 3, **caractérisées en ce que** le volume occupé par les microcapsules, dont la taille de particules est ≤ 6 micromètres, est ≥ 20 %, en particulier ≥ 22 % du volume total des microcapsules.

5. Microcapsules selon au moins l'une des revendications 1 à 4, les microcapsules étant **caractérisées en ce qu'**elles présentent une paroi de capsule constituée d'un aminoplaste.

6. Microcapsules selon au moins l'une des revendications précédentes, les microcapsules étant **caractérisées en ce qu'**elles présentent une paroi de capsule constituée d'une résine méthylée de mélamine-formaldéhyde et/ou d'urée-formaldéhyde et/ou de produits réactionnels d'aldéhydes avec de la thiourée, de la N-alkylurée, de la guanidine, de l'acétoguanamine, de la benzoguanamine, de la capro-guanamine, de la cyanamine, du dicyandiamide et/ou de l'alkyl/arylsulfonamide.

7. Microcapsules selon au moins l'une des revendications précédentes, les microcapsules étant **caractérisées en ce qu'**elles présentent une matière centrale constituée d'au moins une matière hydroinsoluble.

8. Microcapsules selon au moins l'une des revendications précédentes, les microcapsules étant **caractérisées en ce qu'**elles présentent une matière centrale qui comprend une huile parfumée hydrophobe, un pesticide, un biocide, un insecticide, une substance provenant du groupe des répulsifs, un principe actif cosmétique, un colorant ou des mélanges de colorants, des couleurs luminescentes, des azurants optiques, des solvants, des cires, des huiles siliconées, des lubrifiants, des monomères pour polymérisations ou polycondensations, des matières plastiques réactives, par exemple des colles pour mono- ou multicomposants, des composants de peinture, des ignifugeants, des dispersions de pigments dans des solvants organiques, des substances aromatiques et/ou des produits agrochimiques.

9. Microcapsules selon au moins l'une des revendications précédentes, les microcapsules étant **caractérisées en ce qu'**elles présentent un rapport en poids de matière centrale sur la matière de la paroi de capsule qui est compris aux environs de 50-90 à 50-10, en particulier aux environs de 70-80 à 30-20.

10. Suspension de microcapsules selon au moins l'une des revendications 1 à 8 dans un liquide.

11. Suspension selon la revendication 10, **caractérisée en ce que** la proportion en poids des microcapsules est située aux environs de 20 à 60 % en poids, en particulier aux environs de 25 à 50 % en poids, de manière mieux préférée aux environs de 30 à 35 % en poids.

12. Suspension selon au moins l'une des revendications 10 ou 11, la suspension étant **caractérisée en ce qu'**elle présente une viscosité dans la plage de 12 à 1500 mPas.

13. Utilisation des microcapsules selon l'une des revendications 1 à 9 ou de la suspension selon au moins l'une des revendications 10 à 12 en tant que composant de lessives liquides, adoucissants, détergents, poudres de lavage, gels douche, shampooings, déodorants et/ou lotions pour le corps.

14. Utilisation des microcapsules selon la revendication 13 en éliminant au préalable l'eau de la suspension, en particulier par séchage par pulvérisation.
